(19) Europäisches Patentamt
European Patent Office
Office européen des brevets

(11) **EP 4 309 727 A1**

(12) **EUROPEAN PATENT APPLICATION**
published in accordance with Art. 153(4) EPC

(43) Date of publication:
**24.01.2024 Bulletin 2024/04**

(21) Application number: **21931666.8**

(22) Date of filing: **01.09.2021**

(51) International Patent Classification (IPC):
*A61N 2/04* (2006.01)    *H01F 5/00* (2006.01)
*H01F 7/20* (2006.01)

(52) Cooperative Patent Classification (CPC):
**A61N 2/02; H01F 5/00; H01F 7/20**

(86) International application number:
**PCT/JP2021/032204**

(87) International publication number:
**WO 2022/195922 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.03.2021  JP 2021045298**

(71) Applicant: **Adtex Inc.
Gunma 370-1201 (JP)**

(72) Inventor: **MUKAIYAMA Hiroshi
Takasaki-shi, Gunma 370-1201 (JP)**

(74) Representative: **Cabinet Chaillot
16/20, avenue de l'Agent Sarre
B.P. 74
92703 Colombes Cedex (FR)**

(54) **COIL STRUCTURE**

(57)    An object of the present invention is to provide a coil structure capable of increasing a magnetic field generated inside an object with respect to a current flowing through a coil. The coil structure of the present invention is a coil structure in which a conducting wire is wound, and 40 to 100% of its total length is wound on a single conical surface. Preferably, an angle between a generatrix and a central axis of the single conical surface is 48° to 60°.

[Fig. 1]

**Description**

[Technical Field]

This application claims priority to Japanese Patent Application No. 2021-45298, the contents of which are hereby incorporated by reference into the present application.

The present invention relates to a coil structure suitable for generating a magnetic field, for example, inside a human head.

[Background Art]

Transcranial magnetic stimulation (TMS) that generates a magnetic field in a specific portion of the brain for the treatment of depression or the like has been proposed.

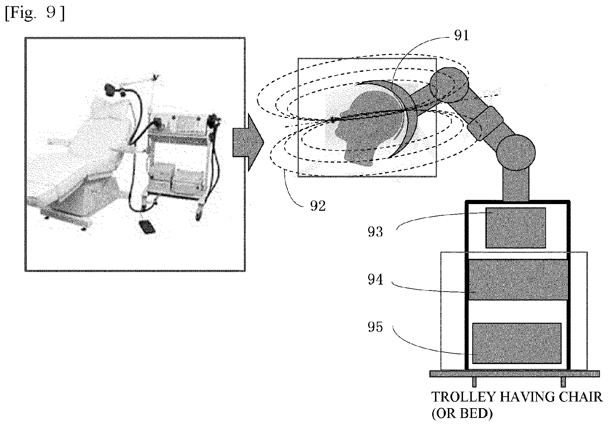

FIG. 9 is an external and conceptual view of a TMS device in the prior art. A headgear 91 is placed on the head of a patient sitting on the sofa of the TMS device, and a magnetic field is emitted from a coil built into the headgear. In this way, a magnetic field can be generated in a specific portion of the brain. A dotted line 92 in the figure conceptually indicates a magnetic field generated from the device, and reference numeral 93 indicates an operation panel, 94 indicates a power driver, and 95 indicates a cooling unit.

[Summary of Invention]

[Technical Problem]

When a coil is energized to generate a magnetic field, it is inevitable that the coil will generate heat. If the coil generates a large amount of heat, a large-scale device is required to cool the coil. An object of the present invention is to provide a coil structure capable of increasing a magnetic field generated inside an object with respect to a current flowing through a coil.

[Solution to Problem]

As a result of intensive studies, the present inventors have accomplished the present invention having the following contents.

[1] A coil structure in which a conducting wire is wound, wherein 40 to 100% of a total length thereof is wound on a single conical surface.
[2] The coil structure according to [1], wherein the single conical surface is configured such that an angle between a generatrix and a central axis thereof is 48° to 60°.
[3] The coil structure according to [1] or [2], which is used by being mounted on a human head.
[4] A coil structure configured by conically winding a conducting wire along a peripheral edge of a spherical body along an axis connecting a point where a predetermined magnetic flux density is to be generated and a point on a surface of the spherical body, wherein in a position where a relationship between a distance (z) between an acting point and a center of a coil and a distance (a) of the peripheral edge of the spherical body from an axis is a $\leq 2^{1/2} \times z$, the conducting wire is wound with a coil diameter that is between a generatrix and the peripheral edge of the spherical body such that an angle between the axis and the generatrix is 48 to 60°.
[5] The coil structure of [4], having a core within at least a portion of the coil structure.

[Effect of the present invention]

According to the present invention, the magnetic field generated inside the conical shape increases with respect to the current flowing through the coil. Therefore, the current flowing through the coil can be made relatively small, so the device for cooling the coil can be simplified.

[Brief Description of Drawings]

[FIG. 1]

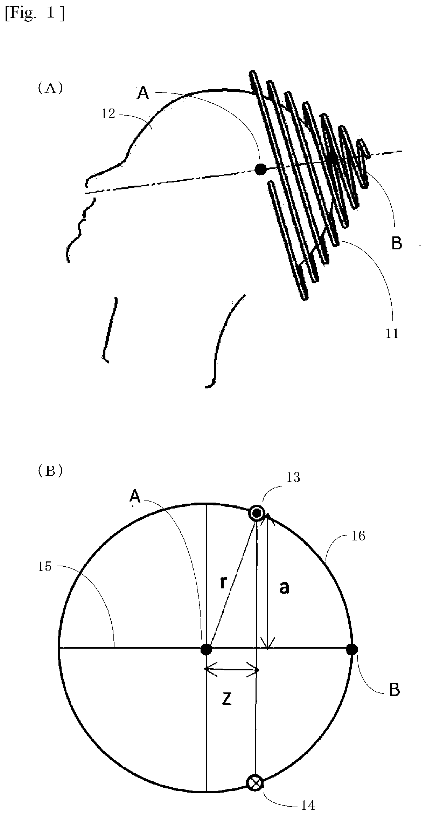

FIG. 1 is a schematic view of the application of a coil according to the present invention to the human head.
[FIG. 2]

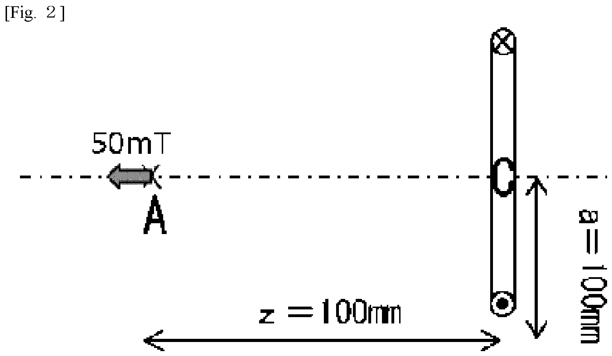

FIG. 2 defines the radius a of the coil and the distance z to an acting target point A.
[FIG. 3]

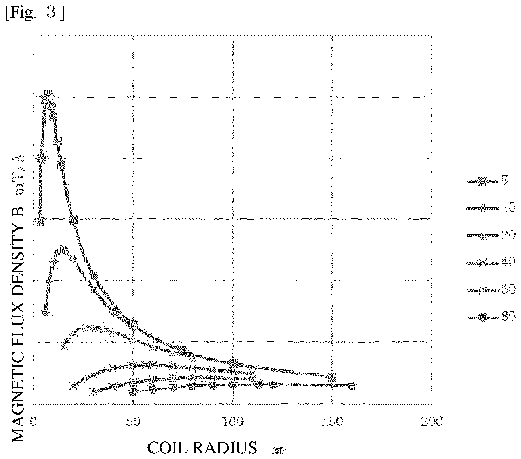

FIG. 3 is a plot of the relationship between the coil radius a and the magnetic flux density B.
[FIG. 4]

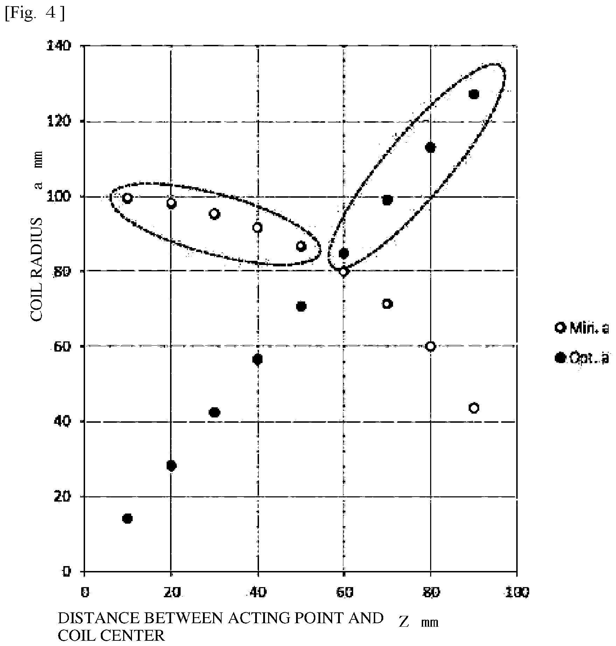

FIG. 4 is a plot (•) of the coil radius that gives the maximum value of the magnetic flux density shown in FIG. 3 with respect to the distance z between the acting target point and the coil center and a plot (○) representing the outer diameter of the acting target.
[FIG. 5]

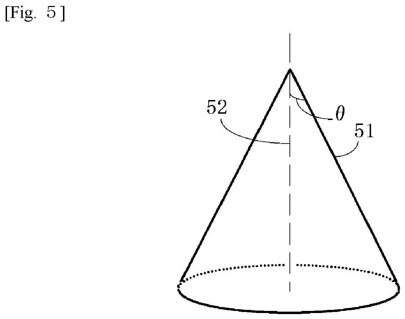

FIG. 5 is an explanatory diagram of a cone.
[FIG. 6-1]

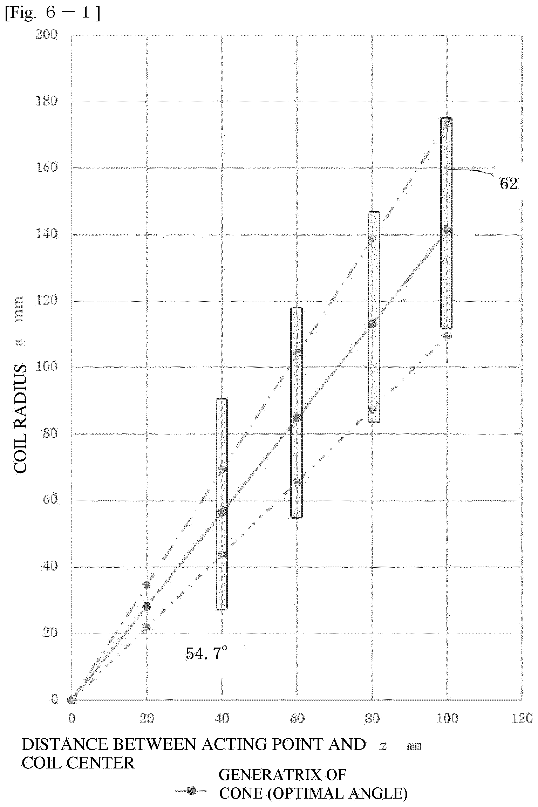

FIG. 6-1 is a layout diagram of a helical coil cross-section.
[FIG. 6-2]

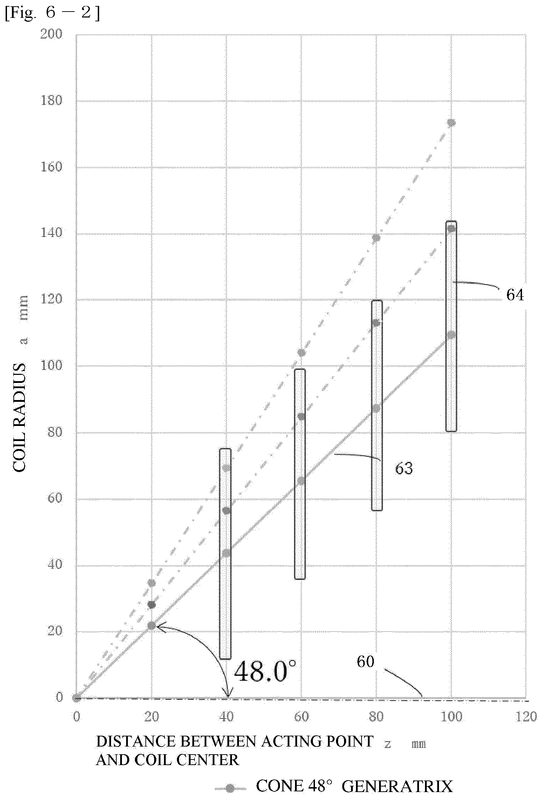

FIG. 6-2 is a layout diagram of a helical coil cross-section.
[FIG. 6-3]

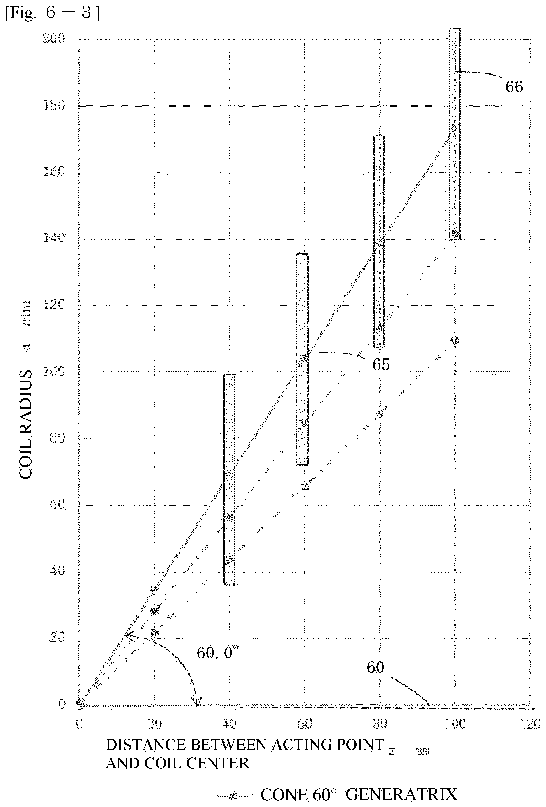

FIG. 6-3 is a layout diagram of a helical coil cross-section.
[FIG. 7]

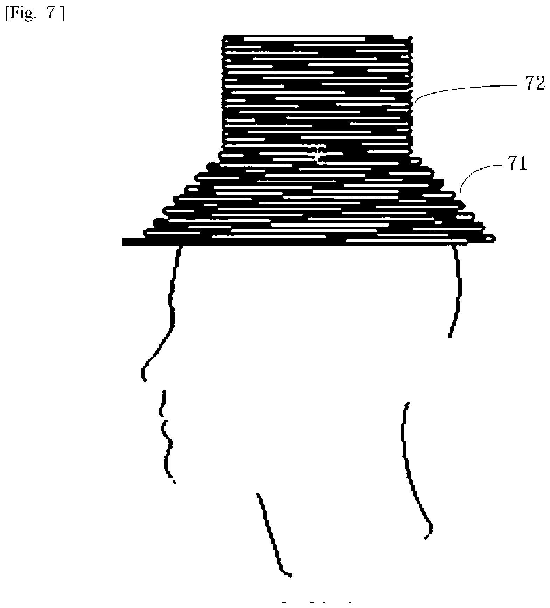

FIG. 7 is a schematic diagram of another embodiment of the coil structure according to the present invention.
[FIG. 8-1]

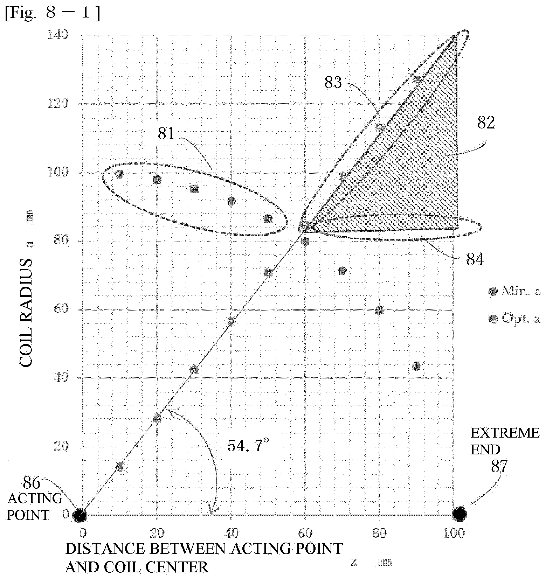

FIG. 8-1 is a partial cross-sectional representation of a coil structure according to the present invention and a plot of a suitable winding diameter for a helical coil.

[FIG. 8-2]

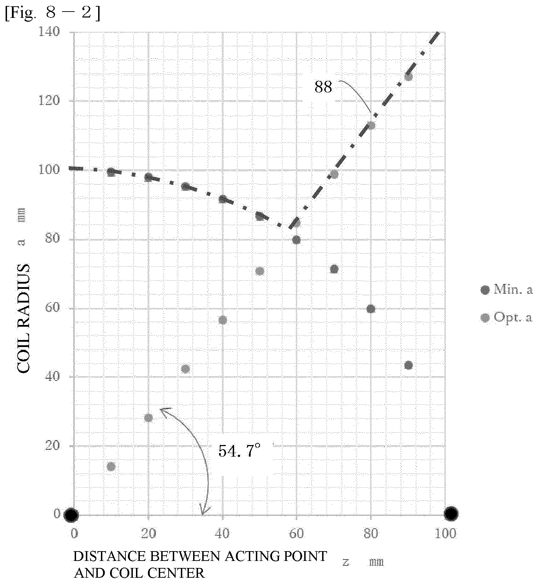

FIG. 8-2 is a partial cross-sectional representation of a coil structure according to the present invention and a plot of a suitable winding diameter for a helical coil.

[FIG. 8-3]

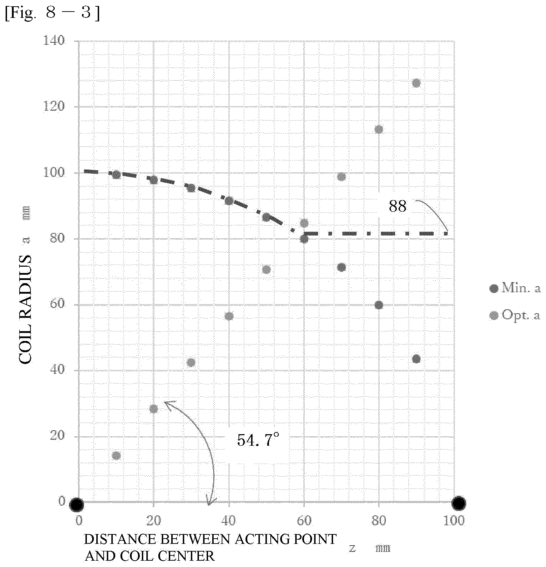

FIG. 8-3 is a partial cross-sectional representation of a coil structure according to the present invention and a plot of a suitable winding diameter for a helical coil.

[FIG. 8-4]

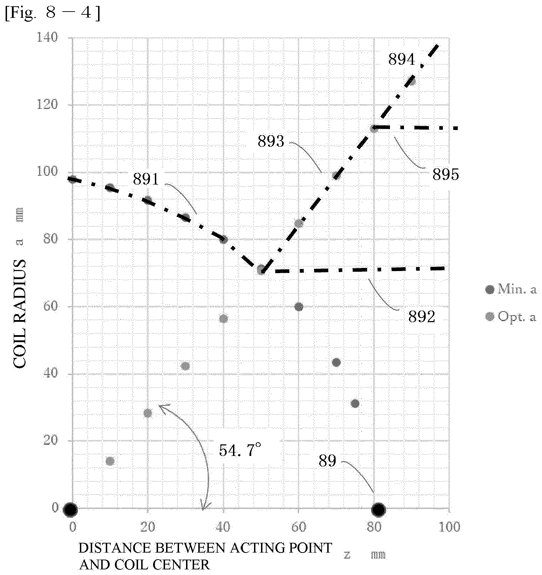

FIG. 8-4 is a partial cross-sectional representation of a coil structure according to the present invention and a plot of a suitable winding diameter for a helical coil.

[FIG. 8-5]

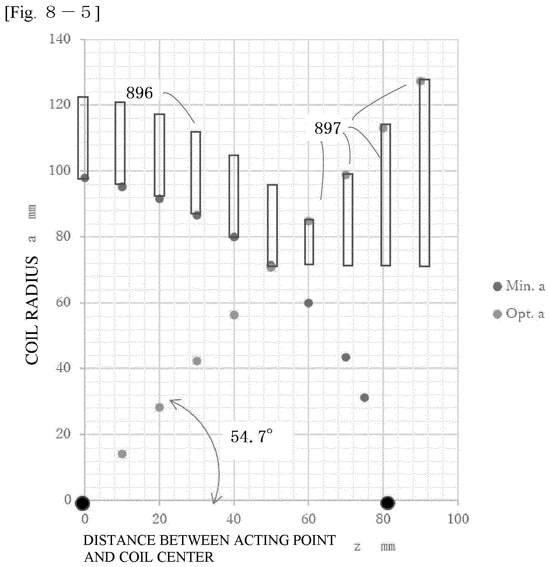

FIG. 8-5 is a partial cross-sectional representation of a coil structure according to the present invention and is a plot of a suitable winding diameter for a helical coil.

[FIG. 9]

FIG. 9 is an external and conceptual view of a TMS device in the prior art.

[Description of Embodiments]

Hereinafter, the present invention will be described in detail with appropriate reference to the drawings. The illustrated embodiments are not intended to limit the present invention, and are only given as examples.

The coil structure of the present invention is particularly useful when it is desired to generate a magnetic field in locations where a conducting wire cannot be installed. For example, a suitable example is when it is desired to generate a magnetic field inside the human head (inside the brain). Other non-limiting examples include application to places that are difficult to excise, such as the chest and abdominal organs of animals such as humans.

According to the present invention, there are no particular restrictions on the conducting wire to be used, the method of connection with the power source, and the like, and conventionally known techniques relating to coil manufacturing can be referred to as appropriate.

FIG. 1 is a schematic view of the application of a coil according to the present invention to the human head. FIG. 1(A) is a schematic external view, FIG. 1(B) shows a simplified cross-section passing through a central axis 15 of a one-turn circular coil, and reference numerals 13 and 14 show cross-sections of the coil itself, indicating that current flows from 14 to the back of the sheet and flows from 13 to the front of the sheet. FIG. 1 is a diagram defining the distance z between a point A, which will be described later, and the center of the coil, and the radius a of the coil. The coil structure of the present invention, composed of conducting wires 11, is conical and in this configuration is attached to the human head 12. A point A in the figure is a location where a predetermined magnetic field is desired to be generated inside the head (inside the brain). A point B in the figure is the intersection of a surface 16 of a target spherical body and the central axis 15 of the coil, and is the extreme end of the head as the target.

The optimal shape of the conical shape formed by the coil is considered with reference to FIG. 1(B). Here, a curve 16 in FIG. (1B) indicates the limit position where the conducting wire can be installed. That is, no conducting wire can be installed inside the circle 16. As a specific example, the curve 16 can be considered to be the surface of a human head. A line 15 is the central axis of the cone formed by the coil structure. The point B mentioned above is located at the intersection of the central axis 15 and the curve 16. The point A is a location where a predetermined magnetic field is desired to be generated as described above. Reference numerals 13 and 14 denote conducting wires that constitute the coil. In the drawing, r is the distance between the point A and the curve 16. When considering the locations of reference numerals 13 and 14 in the conducting wire, a is defined as the distance from the location to the central axis 15, and Z is defined as the distance from the projection point from the reference numeral 13 onto the central axis 15 to the point A.

As a hypothetical example, a case of setting a coil outside a spherical body with a radius of about 100 mm and deriving a coil structure that generates the required magnetic flux density (for example, about 50 mT) at the center will be considered. FIG. 2 is a cross-sectional view including the central axis of the coil. When z is defined as the distance AC between the center C of the coil and the point A where a magnetic field with a predetermined magnetic flux density is desired to be generated, and a is defined as the coil radius, the magnetic flux density B [T] at the point A can be expressed as below.

[Math. 1]

$$B = \mu_0 I \frac{a^2}{2(z^2 + a^2)^{\frac{3}{2}}}$$

Here, I is the current value, $\mu_0$ is the magnetic permeability of the vacuum, and $\mu$ is the relative magnetic permeability of the air in FIG. 2.

FIG. 3 is a plot of the relationship between the radius of the coil and the magnetic flux density B when z, that is, the distance from the center of the coil to the acting point, is used as a parameter. According to this, it is easy to understand by looking at the plots when the positions are separated by 10 mm or 20 mm, for example, and it can be seen that there is an optimum value for the coil radius that can deliver the highest magnetic flux. The optimum value can be expressed by differentiating the above equation, and the relational expression is $a=2^{1/2}\times z$. For example, when z is 60 mm, the radius a is 84.85 mm. Therefore, for the coil diameter, the coil designed with the radius a according to $a=2^{1/2}\times z$ using the distance z to the portion that generates a predetermined magnetic flux is the coil that generates the strongest magnetic flux density.

Therefore, when the parameters a and z are defined as above, the coil radius a that gives the maximum magnetic flux density at a portion of a certain point can be said to be the optimal shape of the coil arranged along $a=2^{1/2}\times z$ on a concentric axis. However, this shape is a conical shape, like a megaphone, with a smaller diameter near the target portion and a larger diameter at a distance, so it may not be possible to place this coil inside the target portion. As a simple example, considering the case where the target object is a sphere, under this condition, according to the design method of the coil radius that satisfies $a=2^{1/2}\times z$, when the distance between the target portion and the coil center is determined, the optimum radius a = 56.6 mm in the case of z = 40 mm, for example. However, considering the above conditions, a radius of 91.5 mm or less corresponds to the inside of the target portion, and the coil cannot be placed, so that the coil is designed with the minimum diameter of the sphere. When the distance to the portion is 80 mm, the optimum diameter is larger than the minimum diameter of the sphere, the coil can be designed with the optimum diameter. To explain this in FIG. 4, the plot (○) surrounded by an ellipse is close to the affected area, so the optimum coil diameter cannot be used, and the coil diameter is designed according to the shape of the sphere. In the plot (•) surrounded by an ellipse, the coil diameter can be designed with the optimum diameter.

As an example of the coil shape resulting from the above consideration, the shape shown in FIG. 7 can be considered. Therefore, with this shape, the magnetic flux density inside 80 mm from the surface of the shape to be irradiated on the coil central axis is calculated as approximately 5.0 [mT] when a high-frequency large current of 100 [A] and 200 [kHz] is applied. For example, when the required magnetic flux density is 40 [mT], it is necessary to stack six to eight coils per unit length. In other words, a coil having the relationship between the coil radius and the distance z from the center of the coil to one point (acting point) on the central axis that generates a predetermined magnetic flux density satisfies the relationship $a=2^{1/2}\times z$ is preferred. In a coil that generates a magnetic field inside an object in which the coil cannot be placed therein, when the distance from the axis of the coil to the surface of the object (z') is less than 1.4 times the distance to the acting point A (z), the distance (z') is used as the coil radius. When the distance (z') is greater than 1.4 times the distance to the acting point (z), 1.4 times the distance (z) is used as the coil radius.

A specific shape of such a coil structure includes a coil structure in which a conducting wire is wound so as to form a single conical surface. In the cone forming the conical surface, the angle between the generatrix and the central axis is preferably 48° to 60°, and the optimum angle is 54.7°. FIG. 5 is an explanatory diagram of a cone, showing a generatrix 51, a central axis 52, and an angle θ between them. In a coil structure, it is not necessary for all the conducting wires to be present on the conical surface for convenience of connection with the power supply, and the like. Specifically, 40 to 100% of the total length of the conducting wires may be on the single conical surface. The process of deriving the optimum angle of 54.7° from the above equation $a=2^{1/2}\times z$ is as follows. That is, the angle is the angle θ calculated as a solution of $\tan\theta=a/z=2^{1/2}$ from the above equation.

FIG. 6-1 is a layout diagram of a helical coil cross-section, showing a state in which the centers of the coil cross-sections 62 are arranged along the angle 54.7° between the generatrix 61 and the coil central axis 60. The actual cross-sectional shape of the coil is rectangular or circular as indicated by 62, and when wound as a coil, the coil has an inner diameter and an outer diameter. The angle between the innermost surfaces of the coil is smaller than 54.7°, and the angle between the outermost surfaces of the coil is larger than 54.7°. When a high-frequency current is actually applied, the current is concentrated on the inner and outer end surfaces of the coil due to the skin effect. In some cases, it may be appropriate to form the generatrix with half of its longitudinal thickness offset inwards or outwards.

For example, if the cross-sections 62, 64, and 66 of the coil are rectangular and the long side of the rectangular cross-section is 64 mm, and the center of the coil cross-section 63 is formed along 54.7°, the angle $\theta_i$ between the central axis and the straight line 64 connecting the coil inner end suface at the end of the position of 100 mm, which corresponds to the maximum diameter of the conical coil, and the cone apex is $\theta_i=48.09°$ from $\tan\theta_i=(100\times2^{1/2}-64/2)/100$. On the other hand, at the outer end surface of the coil, the angle $\theta o$ of the generatrix 65 is 60.03° from $\tan\theta_o=(100\times2^{1/2}+64/2)/100$. Therefore, if the conical coil is formed such that the angle between the generatrix 51 and the coil central axis 52 is set to 48° to 60°, the inner side surface with high current density or the outer side surface can be adjusted to the position of the optimum radius.

FIG. 6-2 shows an example in which the coil is wound so that the center of the cross-section (reference numeral 64) of the terminal coil at the position of 100 mm coincides with the generatrix 63 forming an angle of 48° with the central axis 60. FIG. 6-3 shows an example in which the coil is wound so that the center of the cross-section (reference numeral 66) of the terminal coil at the position of 100 mm coincides with the generatrix 65 forming an angle of 60° with the central axis 60.

FIG. 7 is a schematic diagram of another embodiment of the coil structure according to the present invention. This coil structure has a portion 71 in which a conducting wire is wound on a conical surface and a portion 72 in which a conducting wire is wound on a cylindrical surface. In the embodiment of FIG. 7, approximately 50% of the total length of the wire occupies the portion 71. A core may be incorporated in the hollow portion of the coil at the portion 72. Examples of materials for the core include iron, ferrite, amorphous metal materials, and the like.

FIG. 8-1 is a partial cross-sectional representation of a coil structure in the embodiment of FIG. 7 and is a plot of a suitable winding diameter for a helical coil. Reference numeral 81 in this plot indicates that the conducting wire wound on the conical surface is wound with a coil diameter along the outer shape of the object to which the magnetic field is applied, such as the head. A region indicated by reference numeral 82 represents a region where the coil radius of the conductor wound on the cylindrical surface can be taken. From this plot, the following can be said. The coils are arranged by determining the winding diameter of the coil according to the reference numeral 81 in a portion 60 mm away from the acting point, which is the origin (0, 0) of the graph in FIG. 8-1. In the region 82 from 60 mm to 100 mm, it is most effective to determine the winding diameter according to the optimum winding diameter 83. A stronger magnetic field can be generated by determining the coil diameter in the region 82 surrounded by a parallel line 84 and an optimum line 83 and arranging the core therein. However, although FIG. 8-1 shows the distance from the acting point up to 100 mm, the coil may be wound to a position farther than 100 mm, and the present invention is not limited to the distance of 100 mm. Similarly, FIG. 8-1 does not draw a plot at the position 86 on the object, but the effect is not reduced even when the plot at the position 81 is extended beyond the position of Z=0 and the coil is wound.

As shown in FIG. 8-1, an appropriate coil design method is that, when the distance from the acting point 86 to the extreme end 87 of the object is regarded as 100%, the coil diameter is determined according to the shape of the object in the distance of 60% from the acting point, and the coil diameter is determined between the optimal diameter 83 and the cylindrical winding 84 wound with the same diameter in the distance of 60% or more. As a first example, if the coil is an air-core coil without a core, the coil is wound along a line 88 as shown in FIG. 8-2. As a second example, a cylindrical shape along a line 88 as shown in FIG. 8-3 is the simplest configuration in terms of manufacturing. In addition, as a design method when the maximum depth of the acting point is 80 mm from the end 87 of the object, as shown in FIG. 8-4, the coil may be formed along an outer edge 891 of the object up to a portion at the depth of 48 mm (80×60%). In a portion at a depth of 48 mm or more, the coil may be formed with a constant radius like reference numeral 892, or the coil may be formed along the optimum diameter like reference numerals 893 and 894, or the coil may be wound with the same diameter after the end of the object like reference numerals 893 and 895. Each numerical value is one of examples, and depending on the size of the object, the depth of the acting point, and whether or not the core is built in, the horizontal axis of FIG. 8-1 may be read as a percentage representing the positional relationship between the acting point and the end of the object, and the coil radius may be designed so that the coil is positioned in the region of reference numerals 81 and 82. As another example, as shown in FIG. 8-5, in a region where the distance from the center position of each coil winding and the acting point is 0 to 60%, a coil having a rectangular cross-sectional shape as indicated by reference numeral 896 may be wound along the surface of the object. In the region of 60% or more, the shape of the rectangular cross-section may be gradually changed as indicated by reference numeral 897 so that the inner diameter of the coil winding is kept constant, and the outer diameter of the coil winding follows the optimal plot. At this time, the coil indicated by the coil number 897 may be formed by gradually changing the cross-section of the wire material that is integrated in a spiral shape. Since the induced electromotive force can be used, the coils indicated by the coil number 897 may be independent in a ring shape and may not be connected in series.

In addition, although FIG. 8-5 shows an example in which the coil cross-sections are arranged at a pitch of 10 mm, they may be arranged at a pitch of 5 mm, for example, and the pitch is not limited to the illustrated dimensions.

As described above, according to the present invention, it is possible to efficiently generate a magnetic field with a low current in a place where a conducting wire cannot be installed. Therefore, it is expected that the current required for a desired magnetic field can be reduced, and as a result, heat generation can be suppressed and heat countermeasures can be simplified.

[Reference Signs List]

11    Conducting wire
12    Head
51    Generatrix
52    Central axis

**Claims**

1.    A coil structure in which a conducting wire is wound, wherein 40 to 100% of the total length of the wire is wound on a single conical surface.

2.    The coil structure according to claim 1, wherein the single conical surface is configured such that an angle between a generatrix and a central axis thereof is 48° to 60°.

3. The coil structure according to claim 1 or 2, which is to be mounted on a human head for use.

4. A coil structure configured by conically winding a conducting wire along a peripheral edge of a spherical body along an axis connecting an acting point where a predetermined magnetic flux density is to be generated and a point on a surface of the spherical body, wherein
in a position where a relationship between (a) and (z) is (a) $\leq 2^{1/2} \times$ (z), the conducting wire is wound with a coil diameter that is between a generatrix and the peripheral edge of the spherical body such that an angle between the axis and the generatrix is 48 to 60°, wherein (z) is a distance between said acting point and a center of the coil, and (a) is a distance between the peripheral edge of the spherical body and the axis.

5. The coil structure of claim 4, having a core within at least a portion of the coil structure.

[Fig. 1]

(A)

(B)

[Fig. 2]

[Fig. 3]

[Fig. 4]

DISTANCE BETWEEN ACTING POINT AND Z mm
COIL CENTER

[Fig. 5]

[Fig. 6 − 1]

[Fig. 6 − 2]

COIL RADIUS a mm

DISTANCE BETWEEN ACTING POINT z mm
AND COIL CENTER

CONE 48° GENERATRIX

48.0°

64

63

60

[Fig. 6 – 3]

[Fig. 7 ]

72

71

[Fig. 8 - 1]

[Fig. 8 − 2]

DISTANCE BETWEEN ACTING POINT $z$ mm
AND COIL CENTER

[Fig. 8 − 3]

[Fig. 8 − 4]

[Fig. 8 − 5]

[Fig. 9]

TROLLEY HAVING CHAIR
(OR BED)

## INTERNATIONAL SEARCH REPORT

| International application No. |
|---|
| **PCT/JP2021/032204** |

### A. CLASSIFICATION OF SUBJECT MATTER

*A61N 2/04*(2006.01)i; *H01F 5/00*(2006.01)i; *H01F 7/20*(2006.01)i
FI: A61N2/04; H01F5/00 F; H01F7/20 Z

According to International Patent Classification (IPC) or to both national classification and IPC

### B. FIELDS SEARCHED

Minimum documentation searched (classification system followed by classification symbols)

A61N2/04; H01F5/00; H01F7/20

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | US 5116304 A (CADWELL INDUSTRIES, INC.) 26 May 1992 (1992-05-26) fig. 1-3, columns 3-4 | 1-3 |
| A | | 4-5 |
| X | CN 109091758 A (HENAN ZHENGZHI MEDICAL INSTRUMENT CO., LTD.) 28 December 2018 (2018-12-28) fig. 1-3, paragraphs [0001], [0010]-[0016] | 1-3 |
| A | | 4-5 |
| A | JP 2020-036993 A (NEURONETICS INC) 12 March 2020 (2020-03-12) fig. 1A-2B | 1-5 |
| A | JP 8-52231 A (NAGANO, Akio) 27 February 1996 (1996-02-27) entire text, all drawings | 1-5 |

☐ Further documents are listed in the continuation of Box C.      ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **25 October 2021** | **09 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2021/032204** |

**Box No. III     Observations where unity of invention is lacking (Continuation of item 3 of first sheet)**

This International Searching Authority found multiple inventions in this international application, as follows:

The claims include the following two inventions.

(Invention 1) Claims 1-3
The invention in claims 1-3 relates to a "coil structure formed by winding a wire, wherein 40-100% of the total length thereof is wound around a conical surface."

(Invention 2) Claims 4-5
The invention in claims 4-5 relates to a "coil structure characterized by having a certain magnetic flux density and winding a coil of which the diameter has a distance between a generating line and a peripheral edge of a spherical body, wherein the coil structure is formed along an axial line that connects one point on the surface of the spherical body, and the wire is wound in a conical surface along a peripheral edge of the spherical body, wherein when the relationship between a distance (z) from the point of application to the center of the coil and a distance (a) from an axis core of the peripheral edge of the spherical body satisfies $a \leq 21/2 \times z$, the angle between the axis core and the generating line becomes 48-60°.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2021/032204**

| Patent document cited in search report | | | Publication date (day/month/year) | Patent family member(s) | | | Publication date (day/month/year) |
|---|---|---|---|---|---|---|---|
| US | 5116304 | A | 26 May 1992 | US | 5047005 | A | |
| | | | | US | 4940453 | A | |
| CN | 109091758 | A | 28 December 2018 | (Family: none) | | | |
| JP | 2020-036993 | A | 12 March 2020 | JP | 2017-502784 | A | |
| | | | | US | 2015/0196772 | A1 | |
| | | | | fig. 1A-2B | | | |
| | | | | JP | 2018-83122 | A | |
| | | | | US | 2018/0071544 | A1 | |
| | | | | WO | 2015/109000 | A1 | |
| | | | | CA | 2937158 | A1 | |
| JP | 8-52231 | A | 27 February 1996 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021045298 A